# EUROPEAN PATENT APPLICATION

(11) **EP 2 499 981 A1**
(43) Date of publication of application: **19.09.2012**
(21) Application number: 10829389.5
(22) Date of filing: 10.11.2010
(51) Int. Cl.: A61B 18/12

(54) **ELECTROCAUTERY DEVICE SPECIFIC TO HEPATOTOMY ENHANCED WITH IONIC SOLUTION**

(30) Priority: 10.11.2009 BR PI0904765
(71) Applicant: Universidade Federal De Minas Gerais - UFMG, CEP:31270-901 Belo Horizonte, Minas Gerias (BR)
(72) Inventor: DE REZENDE NETO, João Baptista, 34000-000 Nova Lima - Minas Gerais (BR)
(74) Representative: Winter, Brandl, Fürniss, Hübner, Röss, Kaiser, Polte - Partnerschaft
(86) International application number: PCT/BR2010/000406
(87) International publication number: WO 2011/057376

(57) **Abstract**

The present invention deals with a device capable of cauterizing tissues, especially of the liver. The electrocautery device can reach variable depths for cauterization, through its elongated stems (5). To improve the cauterizing capacity of the device, it uses an ionic solution that passes through a tube (6), inside the handle (2) of the device, which results in a significant reduction of the surgical time and of the perioperative bleeding. Despite being invasive, the invention proposed here did not alter significantly the hepatic transaminase levels which correspond to the preservation of the hepatic tissue adjacent to the area of the removal of the liver.

## Description

### AREA OF THE INVENTION

The present invention deals with a device capable of cauterizing tissues, especially those of the liver. The electrocautery device can reach variable depths of cauterization, through its elongated stems (5). In order to improve the cauterizing capability of the device, it utilizes an ionic solution that passes through a tube (6) in the handle (2) of the device. Despite being invasive, the invention proposed here did not significantly alter the levels of hepatic transaminases, which corresponds to the preservation of the hepatic tissue adjacent to the area of the liver section.

### STATE OF THE ART

Electrocauterization consists of a process of sectioning the tissue using electricity, which is widely used in modern surgery. The procedure is used frequently to staunch the bleeding of small vessels.

Some patents were found in the state of the art th at deal with this subject, for example: request for patent US2006282072, entitled "Electrocautery Instrument", deals with an electrocauterizing instrument characterized by a cauterizing stem and an elongated handle which contains electric lamps, equidistant from the stems. The light gives greater visibility around the area to be cauterized. A DC battery is used inside the handle of the device to provide energy to the light bulbs.

Request for patent US2005070891, entitled "Self-evacuating electrocautery device", describes a device which can be utilized as a scalpel and also as a cautery during a surgical procedure. The instrument comprises an electric blade connected to an electric wire. This blade is attached to a hollow cable in which there is a vacuum pathway to suck up unwanted debris resulting from the cauterization.

Request for Patent EP 1602338, entitled "Self Fluid-assisted electrocautery device", deals with a medical device preferably used for electrocautery. This instrument comprises a cable, a suction tube, and an electrode/blade for electrocautery. The cable is preferably made of nonconductive material, sterilizable and rigid, such as nylon or similar material. The electrode/blade is placed inside the suction tube and the cable. Three connections are made with this invention: a radio frequency terminal, a source of fluid, and a suction tube.

Request for Patent US 2005283150, entitled "Surgical instruments with integrated electrocautery", describes a series of devices, one of which is an electrocautery, for performing surgical procedures in the tissues of the patient's body. The invention includes, in one aspect, a series of devices comprising surgical instruments providing at least one electrode for electrocautery and, on the other hand, provides a method for cutting and cauterizing tissues with the surgical instrument.

Request for Patent US2009182333, entitled "Method and Apparatus for Surgical Electrocautery", deals with a surgical method for electrocautery and a device which cauterizes the cauterization reaches the entire length of the tissue and, also, is capable of delivering sufficient force for electrocautery. This problem is solved using an incompressible fluid contained in the sack or sacks positioned to support one or more electrodes used for electrocautery.

However, none of the technologies cited above presents an electrocautery device such as the present invention, with the capability of reaching variable depths and thus increasing the capacity for cautery.

### PROBLEMS WITH THE STATE OF THE ART

In surgery of the liver, the hepatotomy phase, in which part of the liver is sectioned, is characterized by severe bleeding. Various steps are taken to reduce the bleeding in this part of the operation. The most simple consists of pinching the blood vessels and ligaturing with surgical thread. However, this technique is extremely laborious and time-consuming, which results in more hemorrhaging.

In another aspect, the use of common eletrocautery devices does not provide adequate control of the hemorrhaging by inducing only superficial cautery of the blood vessels. In addition to this, the ultra-sonic technological devices are extremely expensive and require special apparatus in order to function.

### ADVANTAGES OF THE TECHNOLOGY

The invention proposed here presents various advantages over other existing technologies. Among them are:
a) it permits the cauterization of blood vessels, up to a depth of 10 cm;
b) it permits varying the depth of cauterization;
c) the dripping of ionic solution enhances the cauterization of hepatic tissue, in addition to cooling the tissue around the cauterized area;
d) the device permits automatic dripping in the area of the cauterization;
e) the process eliminates the ligaturing of the blood vessels;
f) the device is portable, approximately (12x5 cm);
g) the device is compatible with most electric generators available in surgical centers;
h) the device is inexpensive;
i) it permits using the technique of enucleation of metastases or other tumors of the liver, when indicated, with minimal bleeding without prior vascular control of the hepatic pedicle;
j) the small area of electrocoagulation around the resected tissue does not interfere with the pathological anatomical examination of the surgical portion;
k) it can be used for the removal of tumors of other solid organs when the technique of enucleation of tissue is indicated;
l) it permits removal of the spleen and kidneys in splenectomies and partial nephrectomies.

### LIST OF FIGURES

Figure 1 shows, in a non-limiting way, the front and side view of the device, in addition to a cutaway showing the interior of the handle.
Figure 2 shows, in a non-limiting way, the conducting stems.
Figure 3 shows, in a non-limiting way, the handle, from two different angles of the device, and a longitudinal radial cutaway of the handle.
Figure 4 shows, in a non-limiting way, the interior of the instrument.

### DETAILED DESCRIPTION OF THE INVENTION

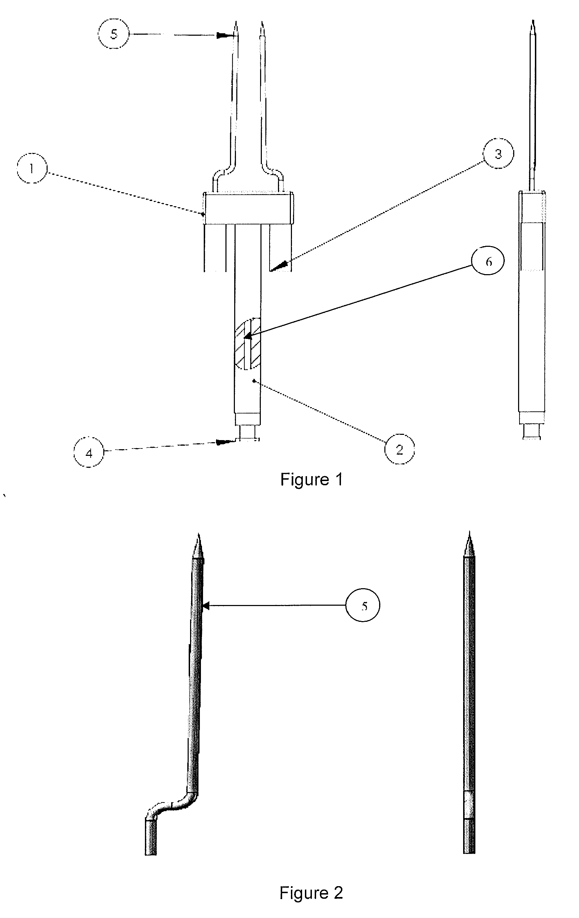

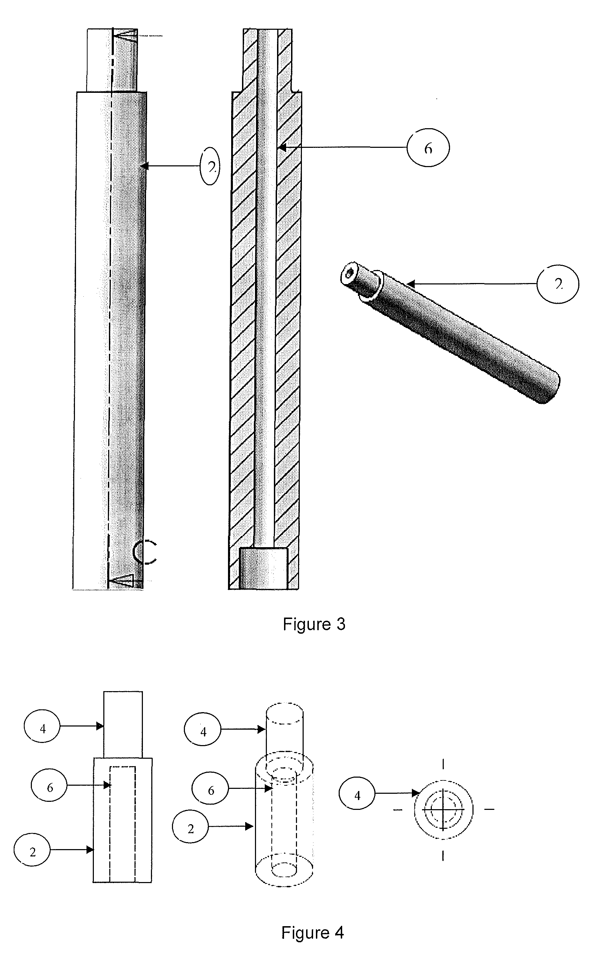

The present invention deals with a device capable of cauterizing tissues, especially of the liver. The electrocautery device can reach variable depths of cauterization, through its elongated stems (5) (Figure 2). In order to improve the cauterizing capability of the device, it utilizes an ionic solution that passes through a tube (6) (Figures 1, 3 and 4) in the handle (2) (Figures 1, 3 and 4) of the device. Any surgical intervention on the hepatic tissue causes damage to the tissues adjacent to the areas being operated on, as can be observed with the use of currently available technologies for the removal of hepatic tissue. One of the forms of evaluating the degree of this damage is the measure of the levels of transaminases in the blood stream. These enzymes increase significantly in the blood when there is injury to the hepatocytes (liver cells). Despite being invasive, the invention proposed here did not alter significantly the levels of hepatic transaminases in cases of hepatectomy (removal of part of the liver) in which it was used, in humans.

Tests in livers *ex vivo* reinforce the importance of the device proposed here in hepatotomies (incisions of parts of the liver). In these tests, the device was shown to be capable of producing precise incisions with minimal macroscopic damage to tissues which are adjacent to the areas of the incisions. The depth of the hepatotomies could also be controlled with precision by a simple increase of pressure on the device at the point of the incision. The design and functionality of the electrocautery device permit the cauterization of blood vessels up to a depth of 10 cm, and the possibility of automatically dripping ionic solution, especially saline, into the area, enhances the capacity of cauterization, resulting in a significant reduction of surgical time and of the trans-operative bleeding.

The present invention can be understood better through the following, non-limiting example:

### Example:

The device was already used in hepatectomies in 15 patients with reproducible and highly promising results, according to the representation in Table 1.

**Table 1 - Values of transaminases monitored in patients with and without the use of the electrocautery device in hepatectomies.**

| **Patient** | **Aspartate Aminotransferase (AST)** | **Alanine Aminotransferase (ALT)** | **AST** | **ALT** | **Electrocautery** |
|---|---|---|---|---|---|
| | **Pre-operative** | **Pre-operative** | **Post-operative** | **Post-operative** | **(Yes/No)** |
| 3 | 32 | 65 | 38 | 72 | No |
| 7 | 25 | 49 | 39 | 69 | No |
| 5* | 112 | 201 | 150 | 310 | No |
| 1 | 23 | 36 | 29 | 42 | Yes |
| 2 | 12 | 27 | 20 | 39 | Yes |
| 4 | 15 | 28 | 32 | 40 | Yes |
| 6 | 18 | 33 | 37 | 52 | Yes |

The monitored values, both pre- and post-operative, were based in the most commonly used transaminases in medical practice, Aspartate Aminotransferase (AST) and Alanine Aminotransferase (ALT). The normal value for the first one is between 5 and 40 units, and for the second one is 7 to 56 units.

Analyzing the results, it is possible to see that in the hepatectomies in which the electrocautery device was used, the variation between pre- and post-operative values of the transaminases of every patient, both for AST and ALT, was less than for the hepatectomies in which the device was not used. In addition to this, the group of patients in which the electrocautery device was used kept the transaminase values within the normal range. Patient 5 was a carrier of Hepatitis C, so the pre- as well as the post-operative values are quite high. Despite being invasive, the invention proposed here did not alter significantly the levels of hepatic transaminases. We currently use the device frequently in hepatectomies in patients with various types of diseases, principally in the removal of hepatic tumors.

## Claims

1. **ELECTROCAUTERY DEVICE**, comprising stems of conductive material (5); hollow cable (2); inner tube (6); connectors (3); base (4); head (1) capable of cauterizing tissues;

2. **ELECTROCAUTERY DEVICE**, according to Claim 1, wherein the interior tube (6) makes possible the automatic dripping of ionic solution, preferably saline solution;

3. **ELECTROCAUTERY DEVICE**, according to Claim 2, wherein the ionic solution enhances the cauterization of the hepatic tissue, in addition to cooling the same tissue around the cauterized area;

4. **ELECTROCAUTERY DEVICE**, according to Claims 1 to 3, comprising portable dimensions from 10 to 15 x 5 to 7 cm;

5. **ELECTROCAUTERY DEVICE**, according to Claims 1 to 4, wherein showing compatibility with most electric generators available in surgical centers;

6. **ELECTROCAUTERY DEVICE**, according to Claims 1 to 5, wherein allowing cauterization of blood vessels up to a depth of 10 cm, as well as varying the depth of cauterization;

7. **ELECTROCAUTERY DEVICE**, according to Claims 1 to 6, wherein the ligaturing of blood vessels is dismissible;

8. **ELECTROCAUTERY DEVICE**, according to Claims 1 to 6, wherein being used specifically in surgical procedures for hepatotomies and hepatectomies;

9. **USE OF THE ELECTROCAUTERY DEVICE**, wherein causing minimal injury to the hepatic tissue which is adjacent to the areas of hepatectomies and hepatotomies, assessed using the serum levels of the hepatic transaminases.
